# NEUE EUROPÄISCHE PATENTSCHRIFT

(11) **EP 1 425 268 B2**
(45) Veröffentlichungstag und Bekanntmachung des Hinweises auf die Entscheidung über den Einspruch: **16.03.2011**
(45) Hinweis auf die Patenterteilung: 27.02.2008
(21) Anmeldenummer: 02797984.8
(22) Anmeldetag: 11.09.2002
(51) Int. Cl.: C07D 207/32

(54) **ORGANISCHE SULFATE ALS IONISCHE FLÜSSIGKEITEN**
ORGANIC SULFATES AS IONIC LIQUIDS
SULFATES ORGANIQUES EN TANT QUE LIQUIDES IONIQUES

(30) Priorität: 17.09.2001 DE 10145747
(43) Veröffentlichungstag der Anmeldung: 09.06.2004
(73) Patentinhaber: Merck Patent GmbH, 64293 Darmstadt (DE)
(72) Erfinder: WASSERSCHEID, Peter, 50829 Köln (DE); BÖSMANN, Andreas, 52068 Aachen (DE); VAN HAL, Roy, NL-6451 HD Schienveld (NL)
(86) Internationale Anmeldenummer: PCT/EP2002/010206
(87) Internationale Veröffentlichungsnummer: WO 2003/022812

(56) Entgegenhaltungen:
- EP-A- 1 182 196
- EP-A- 1 182 197
- WO-A-99/40025
- WO-A1-00/16902
- GB-A- 1 050 791
- GB-A- 1 440 238
- US-B1- 6 245 918
- CHEMICAL ABSTRACTS, vol. 62, no. 8, 12. April 1965 (1965-04-12) Columbus, Ohio, US; abstract no. 9012a, J. KLOUBEK, P. KONDELIK: "Alkyl esters of sulfuric acid and their salts" XP002224891 & CS 111 918 A 15. August 1964 (1964-08-15)
- DATABASE CROSSFIRE BEILSTEIN [Online] Beilstein Institut zur Förderung der Chemischen Wissenschaften, Frankfurt am Main, DE; Database accession no. 4171557 XP002224892 & CORKILL ET AL.: TRANS. FARADAY SOC., Bd. 62, 1966, Seite 987
- DATABASE CROSSFIRE BEILSTEIN [Online] Beilstein Institut zur Förderung der Chemischen Wissenschaften, Frankfurt am Main, DE; .: Database accession no. 3643077 XP002224893 & Z.-J. YU ET AL: J. PHYS. CHEM., Bd. 94, Nr. 9, 1990, Seiten 3675-3681,
- DATABASE CROSSFIRE BEILSTEIN [Online] Beilstein Institut zur Förderung der Chemischen Wissenschaften, Frankfurt am Main, DE; Database accession no. 6709453,6709293,6709083,6708828,6708655 XP002224894 & A. PACKTER, M. DONBROW: J. PHARM. PHARMACOL., Bd. 15, 1963, Seiten 317-324,
- DATABASE CROSSFIRE BEILSTEIN [Online] Beilstein Institut zur Förderung der Chemischen Wissenschaften, Frankfurt am Main, DE; Database accession no. 3643865 XP002224895 & MEGURO, KONDO: NIPPON KAGAKU ZASSHI, Bd. 80, 1959, Seite 818
- DATABASE CROSSFIRE BEILSTEIN [Online] Beilstein Institut zur Förderung der Chemischen Wissenschaften, Frankfurt am Main, DE; Database accession no. 7847922 XP002224896 & V. TOMASIC ET AL.: BER. BUNSEN-GES. PHYS. CHEM., Bd. 101, Nr. 12, 1997, Seiten 1942-1948,
- DATABASE CROSSFIRE BEILSTEIN [Online] Beilstein Institut zur Förderung der Chemischen Wissenschaften, Frankfurt am Main, DE; Database accession no. 6465924 XP002224897 & P. JOKELA ET AL.: J. PHYS. CHEM., Bd. 91, Nr. 12, 1987, Seiten 3291-3298,
- DATABASE CROSSFIRE BEILSTEIN [Online] Beilstein Institut zur Förderung der Chemischen Wissenschaften, Frankfurt am Main, DE; Database accession no. 3814288 XP002224898 & I. M. CUCCOVIA ET AL.: J. CHEM. SOC. PERKIN TRANS. 2, Nr. 9, 2000, Seiten 1896-1907,
- DATABASE CROSSFIRE BEILSTEIN [Online] Beilstein Institut zur Förderung der Chemischen Wissenschaften, Frankfurt am Main, DE; Database accession no. 3862916,3823539,3821574,3808539 XP002224899 & BOLLE, BOURGEOIS: MEM. SERVICES CHIM., Bd. 38, 1953, Seite 159
- DATABASE CROSSFIRE BEILSTEIN [Online] Beilstein Institut zur Förderung der Chemischen Wissenschaften, Frankfurt am Main, DE; Database accession no. 8461725 XP002224900 & A. A. ABRAMZON: ZH. PRIKL. KHIM. (LENINGRAD), Bd. 72, Nr. 4, 1999, Seiten 666-669,
- DATABASE CROSSFIRE BEILSTEIN [Online] Beilstein Institut zur Förderung der Chemischen Wissenschaften, Frankfurt am Main, DE; Database accession no. 3800408 XP002224901 & J. B. S. BONILHA ET AL.: J. PHYS. CHEM., Bd. 93, Nr. 1, 1989, Seiten 367-372,
- PATENT ABSTRACTS OF JAPAN vol. 017, no. 594, 29. Oktober 1993 (1993-10-29) & JP 05 178798 A (KAO CORP.), 20. Juli 1993 (1993-07-20)
- B. GUILLOTEAU-BERTIN ET AL.: "Stereocontrolled Alkylation of Chiral Pyridinium Salt Toward a Short Enantioselective Access to 2-Alkyl- and 2,6-Dialkyl-1,2,5,6-Tetrahydropyridines" EUR. J. ORG. CHEM., Nr. 8, 2000, Seiten 1391-1399, XP002224904

## Beschreibung

Diese Erfindung bezieht sich auf neuartige ionische Flüssigkeiten der generellen Formel [Kation] [R'-SO₄] wobei R' eine verzweigte oder lineare, gesättigte oder ungesättigte, aliphatische oder alicyclische, funktionalisierte oder unfunktionalisierte Kohlenwasserstoffketten mit 3-36 Kohlenstoffatomen darstellt. Diese neuartigen ionischen Flüssigkeiten können z. B. als Lösungsmittel bzw. Lösungsmittelzusätze in chemischen Reaktionen, als Extraktionsmittel oder als Wärmeträger verwendet werden.

### Technischer Zusammenhang der Erfindung

Unter ionischen Flüssigkeiten versteht man allgemein Salze oder Gemische aus Salzen, deren Schmelzpunkte unterhalb 100°C liegen (P. Wasserscheid, W. Keim, Angew. Chem. 2001, 112, 3926). Literaturbekannte Salze dieser Art bestehen aus Anionen wie z. B. Halogenostannaten, Halogenoaluminaten, Hexafluorophosphaten oder Tetrafluoroboraten kombiniert mit substituierten Ammonium-, Phosphonium, Pyridinium- oder Imidazolium-Kationen. Mehrere Veröffentlichungen beschreiben bereits die Verwendung ionischer Flüssigkeiten als Lösungsmittel für chemische Reaktionen (T. Welton, Chem. Rev. 1999, 99, 2071, P. Wasserscheid, W. Keim, Angew. Chem., 2000, 112, 3926). Beispielsweise wurden Hydrierungen von Olefinen mit Rhodium(I)) (P. A. Z. Suarez, J. E. L. Dullius, S. Einloft, R. F. de Souza und J. Dupont, Polyhedron 15/7, 1996, 1217-1219), Ruthenium(II) und Cobalt(II) komplexen (P. A. Z. Suarez, J. E. L. Dullius, S. Einloft, R. F. de Souza und J. Dupont, Inorganica Chimica Acta 255, 1997, 207-209) in ionischen Flüssigkeiten mit Tetrafluoroborat-Anion erfolgreich durchgeführt. Auch die Hydroformylierung von funktionalisierten und unfunktionalisierten Olefinen gelingt mit Rhodium-Katalysatoren in ionischen Flüssigkeiten mit schwach koordinierenden Anionen (z. B. PF₆⁻, BF₄⁻) (Y. Chauvin, L. Mussmann, H. Olivier, European Patent, EP 776880, 1997**;** Y. Chauvin, L. Mussmann, H. Olivier, Angew. Chem., Int. Ed. Engl., 1995, 34, 2698; W. Keim, D. Vogt, H. Waffenschmidt, P. Wasserscheid, J. of Cat., 1999, 186, 481).

Weitere wichtige Einsatzfelder ionischer Flüssigkeiten liegen in ihrer Verwendung als Extraktionsmittel zur Stofftrennung (J. G. Huddleston, H. D. Willauer, R. P. Swatloski, A. E. Visser, R. D. Rogers, Chem. Commun. 1998, 1765-1766; b) A. E. Visser, R. P. Swatlowski, R. D. Rogers, Green Chemistry 2000, 2(1), 1-4) und in ihrer Verwendung als Wärmeträger (M. L. Mutch, J. S. Wilkes, Proceedings of the Eleventh International Symposium on Molten Salts, P. C. Trulove, H. C. De Long, G. R. Stafford and S. Deki (Hrsg.), Proceedings Volume 98-11, The Electrochemical Society, Inc, Pennington, NJ; 1998, S. 254).

WO 00/16902 beschreibt ionische Flüssigkeiten erhältlich durch Reaktion einer organo-Stickstoff- oder -Phosphor- Verbindung, frei von Levois-Saüre-Acidität mit einer Br⌀nsted-Saüre, die als Saürekatalysator oder Lösungsmittel für Saüre-Katalysatoren verwendet werden können.

EP 1 182 196 beschreibt die Synthese von 1-Butyl-3-methylimidazolium butylsulfat.

### Hintergrund und Problemstellung

Auch wenn die Definition für ionische Flüssigkeit auch solche Salze einschließt, deren Schmelzpunkte zwischen Raumtemperatur und 100°C liegen, so ist es doch für viele Anwendungen erforderlich oder wünschenswert, daß die ionischen Flüssigkeiten bereits bei Temperaturen unterhalb von Raumtemperatur flüssig sind.

Zahlreiche Beispiele für solche ionischen Flüssigkeiten sind bekannt, allerdings besitzen diese Systeme in der Regel Halogenidionen wie F, Cl-, Br⁻ oder I⁻ oder solche Anionen, die Halogenatome enthalten. Typische Vertreter der letztgenannten Anionen sind - ohne Anspruch auf Vollständigkeit - [BF₄]⁻, [PF₆]⁻, [CF₃COO]⁻, [CF₃SO₃]⁻, [(CF₃SO₂)₂N]⁻, [AlCl₄]⁻, [Al₂Cl₇]⁻ oder [SnCl₃]⁻. Die Verwendung solcher Halogenatom-haltigen Anionen hat gravierende Einschränkungen für die Anwendbarkeit der entsprechenden ionischen Flüssigkeit zur Folge: a) Die Verwendung dieser Anionen führt zu erheblichen Kosten, da selbst die Alkalisalze dieser Ionen bereits sehr teuer sind; b) Hydrolyseprodukte der Halogenatom-haltigen Anionen führen zu erheblicher Korrosion in Stahl- und z. T. auch Glasreaktoren; c) Die thermische Entsorgung einer "verbrauchten" ionischen Flüssigkeit mit Halogenatom-haltigen Anionen verursacht in der Regel Korrosions- und Umweltprobleme und ist daher kostspielig. Die Entsorgung über den Abbau in einer biologischen Kläranlage wird ebenfalls durch die Anwesenheit von Halogenatom-haltigen Anionen erschwert.

Generell sind daher Halogenatom-freie ionische Flüssigkeiten von besonderem technischem Interesse, die folgende fünf Eigenschaften kombinieren:
a) Schmelzpunkt bzw. Glaspunkt von unter 25 °C;
b) hydrolysestabil in neutraler wäßriger Lösung (pH = 7) bis 80 °C;
c) thermisch zu entsorgen, ohne Bildung problematischer Verbrennungsgase
d) in biologischen Kläranlage abbaubar.
e) Anion als Alkalisalz kommerziell kostengünstig erhältlich.

Unter den nach dem Stand der Technik bekannten Halogenatom-freien ionischen Flüssigkeiten gibt es bisher keine Vertreter, der dieses komplexe technische Anforderungsprofil erfüllen kann. So sind Nitrat-, Nitrit-, Sulfat (J. S. Wilkes, M. J. Zaworotko, J. Chem. Soc. Chem. Commun. 1992, 965) und Benzolsulfonatschmelzen (H. Waffenschmidt, Dissertation, RWTH Aachen 2000) zwar bekannt, diese ionischen Flüssigkeiten besitzen aber Schmelzpunkte über Raumtemperatur. Hydrogensulfate und Hydrogenphosphate reagieren in wäßriger Lösung unter Abspaltung eines oder mehrerer Protonen und bilden saure wäßrige Lösungen. Methylsulfat und Ethylsulfatschmelzen zeigen bereits nach 1h bei 80°C in wässriger Lösung deutliche Hydrolyse unter Bildung von Hydrogensulfatanionen und dem entsprechenden Alkohol (siehe auch Vergleichsbeispiele 1 und 2).

### Unsere Erfindung als Problemlösung

Unsere Erfindung beruht auf der überraschenden Feststellung, daß ionische Flüssigkeiten gemäß Anspruch 1, die aus einer Kombination eines geeigneten organischen Kations - wobei Imidazolium-, Pyridinium- oder Phosphoniumkationen besonders geeignete Beispiele sind - mit Anionen der allgemeinen Formel [R-SO₄] bestehen - wobei R ein linearer oder verzweigter, gesättigter oder ungesättigter, aliphatischer oder alicyclischer, funktionalisierter oder unfunktionalisierter Alkylrest mit 3-36 Kohlenstoffatomen darstellt - sich genau durch die bereits erwähnte, hochinteressante und technisch relevante Eigenschaftskombination auszeichnen: Die neuartigen ionischen Flüssigkeiten gemäß dieser Erfindung weisen zum einen Schmelzpunkte bzw. Glaspunkte von unter 25 °C auf, sie sind ferner hydrolysestabil in neutraler wäßriger Lösung (pH = 7) bis 80 °C. Außerdem sind die ionischen Flüssigkeiten gemäß dieser Erfindung unproblematisch thermisch zu entsorgen, da bei ihrer Verbrennung lediglich CO₂, H₂O und SO₂ gebildet wird. Ein weiterer wesentlicher Vorteil der neuartigen ionischen Flüssigkeiten gemäß dieser Erfindung stellt die Tatsache dar, daß viele Alkalisalze der allgemeinen Formel [Alkalikation] [R-SO₄] - wobei R ein linearer oder verzweigter, funktionalisierter oder unfunktionalisierter, gesättigter oder ungesättigter, aliphatischer oder alicyclischer Alkylrest mit 3-36 Kohlenstoffatomen darstellt - technisch leicht verfügbare Rohstoffe für Waschmittel und für Produkte im Bereich der Kosmetik und Reinigungsmittel sind. Daraus resultiert ein außerordentlich guter Kenntnisstand über die toxikologischen Eigenschaften und das biologische Abbauverhalten der Anionkomponente [RSO₄]. Daraus folgt, daß die Entsorgung der in technischen Anwendungen "verbrauchten" ionischen Flüssigkeiten gemäß dieser Erfindung in biologischen Kläranlagen unproblematisch vollzogen werden kann.

Die technisch hochinteressante Kombination dieser fünf Eigenschaften zeichnet die neuartigen ionischen Flüssigkeiten gemäß dieser Erfindung als ideale Lösungsmittel für stoichiometrische oder katalytische chemische Umsetzungen sowie für ihre Anwendungen als Extraktionsmittel und als Wärmeträger aus.

Besonders bevorzugt lassen sich auch solche ionische Flüssigkeiten gemäß dieser Erfindung herstellen und verwenden, die Gemische von unterschiedlichen Anionen der allgemeinen Formel [RSO₄] enthalten - wobei R ein linearer oder verzweigter, gesättigter oder ungesättigter, aliphatischer oder alicyclischer, funktionalisierter oder unfunktionalisierter Alkylrest mit 3-36 Kohlenstoffatomen darstellt. Diese Systeme lassen sich leicht aus den Gemischen der entsprechenden Alkalisalze erhalten, die technisch verfügbar sind.

Explizit erwähnt seien folgende neuartige ionische Flüssigkeiten gemäß dieser Erfindung sowie ihre Gemische:
1-Ethyl-3-methylimidazolium butylsulfat
1-Ethyl-3-methylimidazolium octylsulfat
1-Ethyl-3-methylimidazolium 2-ethylhexylsulfat
1-Ethyl-3-methylimidazolium dodecylsulfat
1-Butyl-3-methylimidazolium octylsulfat
1-Butyl-3-methylimidazolium 2-ethylhexylsulfat
1-Butyl-3-methylimidazolium dodecylsulfat
1-Hexyl-3-methylimidazolium butylsulfat
1-Hexyl-3-methylimidazolium octylsulfat
1-Hexyl-3-methylimidazolium 2-ethylhexylsulfat
1-Hexyl-3-methylimidazolium dodecylsulfat
1-Octyl-3-methylimidazolium butylsulfat
1-Octyl-3-methylimidazolium octylsulfat
1-Octyl-3-methylimidazolium 2-ethylhexylsulfat
1-Octyl-3-methylimidazolium dodecylsulfat
1-Decyl-3-methylimidazolium butylsulfat
1-Decyl-3-methylimidazolium octylsulfat
1-Decyl-3-methylimidazolium 2-ethylhexylsulfat
1-Decyl-3-methylimidazolium dodecylsulfat
1-Dodecyl-3-methylimidazolium butylsulfat
1-Dodecyl-3-methylimidazolium octylsulfat
1-Dodecyl-3-methylimidazolium 2-ethylhexylsulfat
1-Dodecyl-3-methylimidazolium dodecylsulfat
1-Butyl-pyridinium butylsulfat
1-Butyl-pyridinium octylsulfat
1-Butyl-pyridinium 2-ethylhexylsulfat
1-Butyl-pyridinium dodecylsulfat
Trihexyltetradecylphosphonium butylsulfat
Trihexyltetradecylphosphonium octylsulfat
Trihexyltetradecylphosphonium 2-ethylhexylsulfat
Trihexyltetradecylphosphonium dodecylsulfat

### Beispiele

### Beispiel 1: 1,3-Dimethylimidazoliumoctylsulfat ([MMIM] [OcSO₄])

### Synthese:

Zu einer Lösung von 47.18 g (355.8 mmol) 1,3-Dimethylimidazoliumchlorid ([MMIM] Cl) in 400 ml absolutiertem Methylenchlorid werden 95.00 g (355.8 mmol min.) Natriumoctylsulfat (technische Qualität; Gehalt ≥ 87%) in kleinen Portionen gegeben. Der Ansatz wird 40 Stunden unter Schutzgas gerührt. Der Feststoff wird abfiltriert und mit Methylenchlorid gewaschen. Einengen der organischen Phasen und Trocknen im Hochvakuum liefert 87.55 g [MMIM] [OcSO₄] (285.7 mmol; 80% der theoretischen Ausbeute) in Form einer gelblichen Flüssigkeit.

### NMR:

¹H-NMR (300 MHz, d⁶-DMSO): δ = 8.87 (s, 1H, N-C**H**-N), 7.45, 7.44 (je ein s, je 1H, N-C**H**), 3.87 (mult., 8H, N-C**H**₃, S-O-C**H**₂-), 1.57 (mult., 2H, S-O-CH₂-C**H**₂-), 1.29 (k.B., 10H, S-O-CH₂-CH₂-CH₂-(C**H**₂)₅-), 0.89 (t, J=6.6 Hz, 3H, -CH₂-C**H**₃) ppm.
¹³C-NMR (75 MHz, d⁶-DMSO): δ = 136.7, 122.8, 116.8, 35.1, 30.9, 28.7, 28.4, 25.1, 21.7, 12.8 ppm.

### Viskosität

Das Produkt zeigt Strukturviskosität. Die Viskosität ist stark abhängig von den Meßbedingungen.

### Beispiel 2: 1-n-Butyl-3-methylimidazoliumoctylsulfat ([BMIM] [OcSO₄])

### Darstellung

In 200 ml heißem Wasser werden 84.55 g (0.484 mol) 1-Butyl-3-methylimidazoliumchlorid (BMIM Cl) und 101.1 g (0.379 mol min.) Natriumoctylsulfat (technische Qualität; Gehalt ≥ 87%) gelöst. Das Wasser wird im Vakuum langsam entfernt. Der entstandene Feststoff wird nach Lösen des Ansatzes in Methylenchlorid abfiltriert. Das Filtrat wird mit Wasser gewaschen bis die wäßrige Phase farblos und chloridfrei ist. Die organische Phase wird über Na₂SO₄ getrocknet. Einengen und Trocknen im Hochvakuum liefert 111.0 g (0.319 mmol; 73% der theoretischen Ausbeute bezogen auf Natriumoctylsulfat) einer öligen, gelben Flüssigkeit.

### NMR

¹H-NMR (300 MHz, d⁶-DMSO): δ = 9.16 (s, 1H, N-C**H**-N), 7.80, 7.72 (je s, je 1H, N-C**H**), 4.18 (t, 3J=7.1 Hz, 2H, N-C**H**₂-), 3.86 (s, 3H, N-C**H**₃), 3.71 (t, ³J=6.6 Hz, 2H, S-O-C**H**₂), 3.71 (p, 3J=7.3 Hz, 2H, N-CH₂-C**H**₂-), 1.47 (k.B., 2H, N-CH₂-CH₂-C**H**₂-), 1.22 (mult., 12H,S-O-CH₂-(C**H**₂)₆-), 0.81-0.90 (je tr, je 3H, -C**H**₃) ppm.
13C-NMR (75 MHz, d⁶-DMSO): δ = 136.9, 123.9, 122.6, 66.0, 55.2, 48.8, 36.0, 31.8, 31.6, 29.4, 29.1, 25.9, 22.4, 19.1, 14.2, 13.5 ppm.

### Viskosität

η(20°C)=711 cP

### Beispiel 3: 1-n-Butyl-3-methylimidazoliumlaurylsulfat ([BMIM]

[C₁₂H₂₅SO₄])

### Synthese:

In 50 ml heißem Wasser werden 15.30 g (87.6 mmol) 1-*n*-Butyl-3-methylimidazoliumchlorid (BMIM Cl) und 26.60 g (87.6 mmol min.) Natriumlaurylsulfat (technische Qualität, Gehalt 95-99%) gelöst. Das Wasser wird im Vakuum langsam entfernt. Der entstandene Feststoff wird nach Versetzen des Ansatzes mit Methylenchlorid abfiltriert. Das Filtrat wird mit Wasser gewaschen bis die wäßrige Phase farblos und chloridfrei ist. Die organische Phase wird über Na₂SO₄ getrocknet. Einengen und Trocknen im Hochvakuum liefert 33.40 g Produkt (82.5 mmol; 94% der theoretischen Ausbeute bezogen auf BMIM CI), das als weißer beigefarbener, wachsartiger Feststoff anfällt.
Schmelzpunkt: 44 - 45°C

### NMR:

¹H-NMR (300 MHz, CD₃CN): δ = 8.76 (s, 1H, N-C**H**-N), 7.43, 7.40 (zwei s, je 1H, N-C**H**₃), 4.17 (t, J=7.3 Hz, 2H, N-C**H**₂), 3.87 (s, 3H, N-CH₃), 3.83 (t, J=6.6 Hz, 2H, S-O-C**H**₂-), 1.84 (mult, 2H, N-CH₂-C**H**₂-), 1.58 (mult., 2H, S-O-CH₂-C**H**₂-), 1.40-1.25 (mult., 20H, S-O-CH₂-CH₂-(C**H**₂)₉-; N-CH₂-CH₂-C**H**₂-), 1.00-0.85 (t, je 3H, -C**H**₃) ppm.
¹³C-NMR (75 MHz, CD3CN): δ = 136.2, 123.3, 121.9, 65.9, 48.9-48.7, 35.4, 31.3, 29.1-28.7, 25.5, 22.1, 18.6, 13.1, 12.4 ppm.

### Hydrolyseversuche

### Beispiel 4: Hydrolyseversuch mit 1-n-Butyl-3-methylimidazoliumoctylsulfat ([BMIM] [C₈H₁₇SO₄])

5 g der ionischen Flüssigkeit 1-*n*-Butyl-3-methylimidazoliumoctylsulfat ([BMIM] [C₈H₁₇SO₄]) werden mit 5 ml Wasser versetzt und auf 80 °C erhitzt. Im Abstand von 10 min werden Proben aus der Reaktionslösung genommen und pH-Messungen durchgeführt. Auch nach 2h bei 80°C ist die Reaktionslösung pH-neutral, was darauf schließen läßt, daß unter diesen Reaktionsbedingungen keine hydrolytische Zersetzung der ionischen Flüssigkeit auftritt.

### Vergleichsbeispiel 1: Hydrolyseversuch mit 1-n-Butyl-3-methylimidazoliummethylsulfat ([BMIM] [CH₃SO₄])

5 g der ionischen Flüssigkeit 1-*n*-Butyl-3-methylimidazoliummethylsulfat ([BMIM] [CH₃SO₄]) werden mit 5 ml Wasser versetzt und auf 80 °C erhitzt. Im Abstand von 10 min werden Proben aus der Reaktionslösung genommen und pH-Messungen durchgeführt. Bereits ab der ersten Messung zeigt ein rasches Absinken des pH-Werts bis auf pH 1-2. Dies läßt darauf schließen, daß unter diesen Reaktionsbedingungen eine hydrolytische Zersetzung der ionischen Flüssigkeit auftritt. Dabei wird Methanol und das saure Hydrogensulfatanion freigesetzt.

### Vergleichsbeispiel 2: Hydrolyseversuch mit 1-Ethyl-3-methylimidazoliumethylsulfat ([EMIM] [C₂H₅SO₄])

5 g der ionischen Flüssigkeit 1-Ethyl-3-methylimidazoliumethylsulfat ([EMIM] [C₂H₅SO₄]) werden mit 5 ml Wasser versetzt und auf 80 °C erhitzt. Im Abstand von 10 min werden Proben aus der Reaktionslösung genommen und pH-Messungen durchgeführt. Bereits ab der ersten Messung zeigt ein rasches Absinken des pH-Werts bis auf pH 1-2. Dies läßt darauf schließen, daß unter diesen Reaktionsbedingungen eine hydrolytische Zersetzung der ionischen Flüssigkeit auftritt. Dabei wird Ethanol und das saure Hydrogensulfatanion freigesetzt.

## Patentansprüche

1. Verwendung einer ionische Flüssigkeit der generellen Formel [Kation] [R'-SO₄], wobei R' eine lineare oder verzweigte, gesättigte oder ungesättigte, aliphatische oder alicyclische, unfunktionalisierte oder mit einer oder mehreren Gruppen X funktionalisierte Alkylgruppe mit 3-36 Kohlenstoffatomen darstellt und X eine -OH, -OR", -COOH, -COOR", -NH₂, -SO₄, -F, -Cl, -Br, -I oder -CN -Gruppe ist, wobei R" eine verzweigte oder lineare Kohlenwasserstoffkette mit 1-12 Kohlenstoffatomen repräsentiert, als Lösungsmittel für stöchiometrische oder katalytische chemische Umsetzungen, Extraktionsmittel, PhasentransferKatalysator oder Wärmeträger, wobei das verwendete [Kation] ein
- Phosphonium-Kation der allgemeinen Formel
[PR¹R²R³R]⁺,
- Imidazolium-Kation der allgemeinen Formel 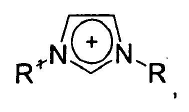 darstellt,
wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyridinium-Kationen der allgemeinen Formel 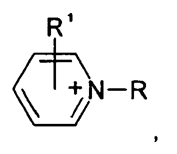 wobei der Pyridin-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyrazolium-Kationen der allgemeinen Formel 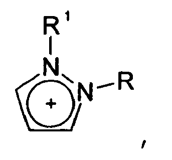 wobei der Pyrazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- und Triazolium-Kationen der allgemeinen Formel 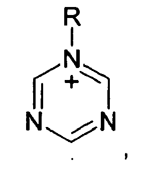 wobei der Triazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen, darstellt und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
und der Rest R ausgewählt ist aus
- linearen oder verzweigten, gesättigten oder ungesättigten, aliphatischen oder alicyclischen Alkylgruppen mit 1 bis 20 Kohlenstoffatomen.

2. Verwendung gemäß Anspruch 1, wobei die ionische Flüssigkeit ein Anion der Summenformel [C₄H₉SO₄], [C₈H₁₇SO₄] oder [C₁₂H₂₅SO₄] trägt.

3. Ionische Flüssigkeiten ausgewählt aus der Gruppe bestehend aus
1-Ethyl-3-methylimidazolium butylsulfat
1-Ethyl-3-methylimidazolium octylsulfat
1-Ethyl-3-methylimidazolium 2-ethylhexylsulfat
1-Ethyl-3-methylimidazolium dodecylsulfat
1-Butyl-3-methylimidazolium octylsulfat
1-Butyl-3-methylimidazolium 2-ethylhexylsulfat
1-Butyl-3-methylimidazolium dodecylsulfat
1-Hexyl-3-methylimidazolium butylsulfat
1-Hexyl-3-methytimidazolium octylsulfat
1-Hexyl-3-methylimidazolium 2-ethylhexylsulfat
1-Hexyl-3-methylimidazolium dodecylsulfat
1-Octyl-3-methylimidazolium butylsulfat
1-Octyl-3-methylimidazolium octylsulfat
1-Octyl-3-methylimidazolium 2-ethylhexylsulfat
1-Octyl-3-methylimidazolium dodecylsulfat
1-Decyl-3-methylimidazolium butylsulfat
1-Decyl-3-methylimidazolium octylsulfat
1-Decyl-3-methylimidazolium 2-ethylhexylsulfat
1-Decyl-3-methylimidazolium dodecylsulfat
1-Dodecyl-3-methylimidazolium butylsulfat
1-Dodecyl-3-methylimidazolium octylsulfat
1-Dodecyl-3-methylimidazolium 2-ethylhexylsulfat
1-Dodecyl-3-methylimidazolium dodecylsulfat
1-Butyl-pyridinium butylsulfat
1-Butyl-pyridinium octylsulfat
1-Butyl-pyridinium 2-ethylhexylsulfat
1-Butyl-pyridinium dodecylsulfat
Trihexyltetradecylphosphonium butylsulfat
Trihexyltetradecylphosphonium octylsulfat
Trihexyltetradecylphosphonium 2-ethylhexylsulfat
Trihexyltetradecylphosphonium dodecylsulfat.

## Claims

1. Use of an ionic liquid of the general formula [cation] [R'-SO₄], where R' represents a linear or branched, saturated or unsaturated, aliphatic or alicyclic alkyl group having 3-36 carbon atoms which is unfunctionalised or functionalised with one or more groups X, and X is an -OH, -OR", -COOH, -COOR", -NH₂, -SO₄, -F, -Cl, -Br, -I or -CN group, where R" represents a branched or linear hydrocarbon chain having 1-12 carbon atoms, as solvent for stoichiometric or catalytic chemical reactions, extractant, phase-transfer catalyst or heat-transfer medium, where the [cation] used represents a
- phosphonium cation of the general formula
[PR¹R²R³R]⁺,
- imidazolium cation of the general formula 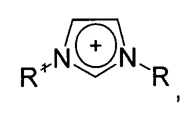 where the imidazole ring may be substituted by at least one group selected from C₁-C₆-alkyl, C₁-C₆-alkoxy, C₁-C₆-aminoalkyl, C₅-C₁₂-aryl or C₅-C₁₂-aryl-C₁-C₆-alkyl groups,
- pyridinium cations of the general formula 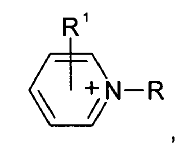 where the pyridine ring may be substituted by at least one group selected from C₁-C₆-alkyl, C₁-C₆-alkoxy, C₁-C₆-aminoalkyl, C₅-C₁₂-aryl or C₅-C₁₂-aryl-C₁-C₆-alkyl groups,
- pyrazolium cations of the general formula 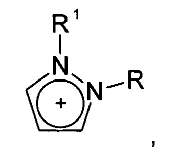 where the pyrazole ring may be substituted by at least one group selected from C₁-C₆-alkyl, C₁-C₆-alkoxy, C₁-C₆-aminoalkyl, C₅-C₁₂-aryl or C₅-C₁₂-aryl-C₁-C₆-alkyl groups,
- and triazolium cations of the general formula 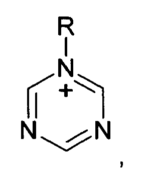 where the triazole ring may be substituted by at least one group selected from C₁-C₆-alkyl, C₁-C₆-alkoxy, C₁-C₆-aminoalkyl, C₅-C₁₂-aryl or C₅-C₁₂-aryl-C₁-C₆-alkyl groups, and the radicals R¹, R², R³, independently of one another, are selected from the group consisting of
- hydrogen;
- linear or branched, saturated or unsaturated, aliphatic or alicyclic alkyl groups having 1 to 20 carbon atoms;
and the radical R is selected from
- linear or branched, saturated or unsaturated, aliphatic or alicyclic alkyl groups having 1 to 20 carbon atoms.

2. Use according to Claim 1, where the ionic liquid carries an anion of the empirical formula [C₄H₉SO₄], [C₈H₁₇SO₄] or [C₁₂H₂₅SO₄].

3. Ionic liquids selected from the group consisting of
1-ethyl-3-methylimidazolium butylsulfate
1-ethyl-3-methylimidazolium octylsulfate
1-ethyl-3-methylimidazolium 2-ethylhexylsulfate
1-ethyl-3-methylimidazolium dodecylsulfate
1-butyl-3-methylimidazolium octylsulfate
1-butyl-3-methylimidazolium 2-ethylhexylsulfate
1-butyl-3-methylimidazolium dodecylsulfate
1-hexyl-3-methylimidazolium butylsulfate
1-hexyl-3-methylimidazolium octylsulfate
1-hexyl-3-methylimidazolium 2-ethylhexylsulfate
1-hexyl-3-methylimidazolium dodecylsulfate
1-octyl-3-methylimidazolium butylsulfate
1-octyl-3-methylimidazolium octylsulfate
1-octyl-3-methylimidazolium 2-ethylhexylsulfate
1-octyl-3-methylimidazolium dodecylsulfate
1-decyl-3-methylimidazolium butylsulfate
1-decyl-3-methylimidazolium octylsulfate
1-decyl-3-methylimidazolium 2-ethylhexylsulfate
1-decyl-3-methylimidazolium dodecylsulfate
1-dodecyl-3-methylimidazolium butylsulfate
1-dodecyl-3-methylimidazolium octylsulfate
1-dodecyl-3-methylimidazolium 2-ethylhexylsulfate
1-dodecyl-3-methylimidazolium dodecylsulfate
1-butylpyridinium butylsulfate
1-butylpyridinium octylsulfate
1-butylpyridinium 2-ethylhexylsulfate
1-butylpyridinium dodecylsulfate
trihexyltetradecylphosphonium butylsulfate
trihexyltetradecylphosphonium octylsulfate
trihexyltetradecylphosphonium 2-ethylhexylsulfate
trihexyltetradecylphosphonium dodecylsulfate.

## Revendications

1. Utilisation d'un liquide ionique de formule générale [cation] [R'-SO₄], où R' représente un groupement alkyle aliphatique ou alicyclique, linéaire ou ramifié, saturé ou insaturé, ayant 3-36 atomes de carbone qui est non fonctionnalisé ou fonctionnalisé par un ou plusieurs groupements X, et X est un groupement -OH, -OR", -COOH, -COOR", -NH₂, -SO₄, -F, -Cl, -Br, -I ou -CN, où R" représente une chaîne hydrocarbonée linéaire ou ramifiée ayant 1-12 atomes de carbone, comme solvant pour des réactions chimiques stoechiométriques ou catalytiques, solvant d'extraction, catalyseur de transfert de phase ou agent de transfert de chaleur, où le [cation] utilisé représente
- un cation phosphonium de formule générale
[PR¹R²R³R]⁺,
- un cation imidazolium de formule générale 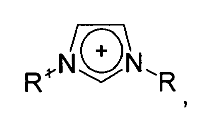 où le cycle imidazole peut être substitué par au moins un groupement choisi parmi des groupements C₁-C₆-alkyle, C₁-C₆-alcoxy, C₁-C₆-aminoalkyle, C₅-C₁₂-aryle ou C₅-C₁₂-aryl-C₁-C₆-alkyle,
- des cations pyridinium de formule générale 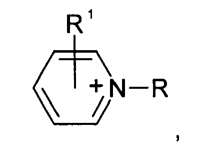 où le cycle pyridine peut être substitué par au moins un groupement choisi parmi des groupements C₁-C₆-alkyle, C₁-C₆-alcoxy, C₁-C₆-aminoalkyle, C₅-C₁₂-aryle ou C₅-C₁₂-aryl-C₁-C₆-alkyle,
- des cations pyrazolium de formule générale 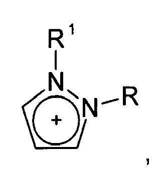 où le cycle pyrazole peut être substitué par au moins un groupement choisi parmi des groupements C₁-C₆-alkyle, C₁-C₆-alcoxy, C₁-C₆-aminoalkyle, C₅-C₁₂-aryle ou C₅-C₁₂-aryl-C₁-C₆-alkyle,
- et des cations triazolium de formule générale 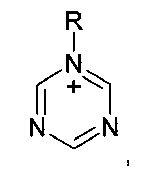 où le cycle triazole peut être substitué par au moins un groupement choisi parmi des groupements C₁-C₆-alkyle, C₁-C₆-alcoxy, C₁-C₆-aminoalkyle, C₅-C₁₂-aryle ou C₅-C₁₂-aryl- C₁-C₆-alkyle, et les radicaux R¹, R², R³, indépendamment les uns des autres, sont choisis dans le groupe constitué par
- hydrogène;
- des groupements alkyle aliphatiques ou alicycliques, linéaires ou ramifiés, saturés ou insaturés, ayant de 1 à 20 atomes de carbone;
et le radical R est choisi parmi
- des groupements alkyle aliphatiques ou alicycliques, linéaires ou ramifiés, saturés ou insaturés, ayant de 1 à 20 atomes de carbone.

2. Utilisation selon la revendication 1, dans laquelle le liquide ionique porte un anion de formule empirique [C₄H₉SO₄], [C₈H₁₇SO₄] ou [C₁₂H₂₅SO₄].

3. Liquides ioniques choisis dans le groupe constitué par
le butylsulfate de 1-éthyl-3-méthylimidazolium,
l'octylsulfate de 1-éthyl-3-méthylimidazolium,
le 2-éthylhexylsulfate de 1-éthyl-3-méthylimidazolium,
le dodécylsulfate de 1-éthyl-3-méthylimidazolium,
l'octylsulfate de 1-butyl-3-méthylimidazolium,
le 2-éthylhexylsulfate de 1-butyl-3-méthylimidazolium,
le dodécylsulfate de 1-butyl-3-méthylimidazolium,
le butylsulfate de 1-hexyl-3-méthylimidazolium,
l'octylsulfate de 1-hexyl-3-méthylimidazolium,
le 2-éthylhexylsulfate de 1-hexyl-3-méthylimidazolium,
le dodécylsulfate de 1-hexyl-3-méthylimidazolium,
le butylsulfate de 1-octyl-3-méthylimidazolium,
l'octylsulfate de 1-octyl-3-méthylimidazolium,
le 2-éthylhexylsulfate de 1-octyl-3-méthylimidazolium,
le dodécylsulfate de 1-octyl-3-méthylimidazolium,
le butylsulfate de 1-décyl-3-méthylimidazolium,
l'octylsulfate de 1-décyl-3-méthylimidazolium,
le 2-éthylhexylsulfate de 1-décyl-3-méthylimidazolium,
le dodécylsulfate de 1-décyl-3-méthylimidazolium,
le butylsulfate de 1-dodécyl-3-méthylimidazolium,
l'octylsulfate de 1-dodécyl-3-méthylimidazolium,
le 2-éthylhexylsulfate de 1-dodécyl-3-méthylimidazolium,
le dodécylsulfate de 1-dodécyl-3-méthylimidazolium,
le butylsulfate de 1-butylpyridinium,
l'octylsulfate de 1-butylpyridinium,
le 2-éthylhexylsulfate de 1-butylpyridinium,
le dodécylsulfate de 1-butylpyridinium,
le butylsulfate de trihexyltétradécylphosphonium,
l'octylsulfate de trihexyltétradécylphosphonium,
le 2-éthylhexylsulfate de trihexyltétradécylphosphonium,
le dodécylsulfate de trihexyltétradécylphosphonium.
